# FASCICULE DE BREVET EUROPEEN

(11) **EP 1 825 818 B1**
(45) Date de publication et mention de la délivrance du brevet: **13.05.2009**
(21) Numéro de dépôt: 07101048.2
(22) Date de dépôt: 23.01.2007
(51) Int. Cl.: A61B 17/06

(54) **Fil chirurgical et procede de fabrication dudit fil**
Chirurgischer Faden und Verfahren zu seiner Herstellung
Surgical thread and method of producing the same

(30) Priorité: 23.02.2006 FR 0601623
(43) Date de publication de la demande: 29.08.2007
(73) Titulaire: Cousin Biotech, 59117 Wervicq Sud (FR)
(72) Inventeur: Solecki, Gilles, 59390, Lannoy (FR); Semeria, Eric, 108, Ambanja (MG)
(74) Mandataire: Hennion, Jean-Claude

(56) Documents cités:
- EP-A1- 1 075 843
- GB-A- 2 159 846
- US-A- 3 123 077
- US-A- 4 510 934
- US-A1- 2004 069 402
- US-B1- 6 289 700

## Description

La présente invention a pour objet un fil chirurgical et un procédé de fabrication permettant notamment l'obtention du dit fil.

L'utilisation de fil chirurgical, notamment pour réunir des tissus souples du corps humain, que ce soit en interne ou en externe est connue.

Afin de proposer au patient des fils chirurgicaux, notamment de suture, en adéquation au mieux avec la pathologie, en tenant compte de leur confort et à moindre coût, différentes configurations de fils sont proposées sur le marché. Les fils chirurgicaux doivent également être faciles à manipuler, à ajuster et à mettre en place par le chirurgien.

On connaît déjà un fil chirurgical dans EP 1 075 843 B1, utilisé dans le domaine de la chirurgie esthétique. Ce fil, de section pleine est réalisé en matériau métallique, polymère ou biologique et comporte une succession de barbes coniques faisant saillie et ayant des extrémités pointues. L'inclinaison des barbes est alternée séquentiellement.

On connaît d'après le document GB 2.159.846A, un élément chirurgical, tel qu'un élément de suture ou un ligament, comprenant une âme multifilamentaires sur laquelle sont tressés plusieurs fils.

On connaît également des monofilaments, de type polypropylène ayant un diamètre égal à 0,34 mm et comportant des fentes à sa surface, commercialisés par la société Assut Europe sous le nom APTOS® pour remonter les tissus du visage qui s'affaissent. Les fils chirurgicaux précités sont rigides et peu élastiques de part leur présentation sous forme d'un « bloc » formé par un monofilament. Par ailleurs, étant de section pleine, le tissu conjonctif accroche difficilement aux aspérités supportées par le fil. De plus, ces dernières sont peu nombreuses, rigides et blessantes pour les tissus environnants.

La présente invention a pour objet de proposer un fil chirurgical, notamment de suture, amélioré en répondant notamment aux inconvénients précités et s'adaptant à des domaines d'application chirurgicale variés ainsi qu'un procédé de fabrication du dit fil chirurgical.

Le fil chirurgical de la présente invention est caractérisé en ce qu'il a une structure maillée, se présentant sous la forme d'une succession continue de mailles, bloquées par intermittence par des tronçons de fil de blocage.

On comprend par le terme tronçon de fil de blocage une longueur déterminée d'un fil unitaire qui contribue éventuellement à la fixation de mailles et qui en assure le blocage, c'est-à-dire qui évite le détricotage des mailles.

Le fil chirurgical de l'invention n'a donc pas une section pleine, puisque sa section est occupée par plusieurs fils unitaires qui vont permettre au tissu conjonctif lors de sa prolifération de pénétrer entre les dits fils et donc de s'accrocher solidement au fil chirurgical. Par ailleurs, la forme arrondie des mailles n'est pas blessante pour les tissus environnants.

De plus, grâce à la capacité de déformation des mailles lorsque le fil chirurgicalest mis sous tension, son élasticité est améliorée par rapport à un fil chirurgical se présentant sous forme d'un monofilament.

Dans une variante de réalisation, les tronçons de fils de blocage ont leurs extrémités libres qui viennent en saillie au-delà, de façon séquentielle, de la section occupée par la succession continue de mailles.

On désigne, dans la suite de la description, par le terme projections, les extrémités libres des fils de blocage qui sont extérieures à la section occupée par la succession continue de mailles.

Les dites projections sont réparties, sur la longueur du fil chirurgical, selon des intervalles réguliers qui sont définis par un nombre déterminé de mailles.

Chaque projection dépasse de la section occupée par la succession de mailles, d'une longueur (I) qu'il est possible de faire varier en fonction des applications réservées au fil chirurgical.

Lorsque les projections ont une longueur (I) très faible, le fil chirurgical apparaît à l'oeil nu comme une simple succession continue de mailles, de surface sensiblement lisse.

Lorsque les projections ont une longueur (I) plus importante, par exemple jusqu'à 5 mm, elles constituent autant de points d'accrochage permettant de stabiliser le fil chirurgical sur les tissus environnants et de favoriser le développement des tissus conjonctifs.

Il est possible de faire varier le volume occupé par le fil chirurgical en ajustant notamment la longueur et le nombre des projections, la taille des mailles, le titrage et le nombre des fils unitaires. Ceci est avantageux, par exemple enchirurgie esthétique en comblement des rides.

Dans une variante de réalisation, le fil chirurgical comporte au moins un premier ensemble de projections, dans lequel chaque projection est inclinée dans une même première direction et éventuellement au moins un second ensemble de projections, dans lequel chaque projection est inclinée dans une même seconde direction, opposée à la première.

La présence de projections inclinées dans des directions opposées, permet de combiner dans le même fil chirurgical deux fonctions, à savoir d'une part la glissance qui facilite par exemple le passage du fil chirurgical dans une aiguille et/ou l'insertion du fil chirurgical dans les tissus du corps humains et d'autre part l'adhérence ou l'accrochage sur les tissus environnants. Chaque projection a une direction générale qui forme avec l'axe longitudinal (XX') du fil de suture un angle (α) qui peut aussi avoir de l'importance selon l'application du fil de suture. De préférence pour la chirurgie esthétique, l'angle (α) est compris entre 15° et 45°.

Les fils unitaires du fil de suture peuvent être des fils monofilaments ou multifilaments, pris notamment parmi les familles de polymères suivantes : polypropylène et poly (α-hydroxyesters) : acide polylactique de forme L(PLLA) ou D (PLDA) et/ou notamment parmi les familles de métaux suivantes titane, alliage titane - nickel, or, acier inoxydable.

Si le fil chirurgical ne nécessite pas d'être résorbable, on choisira de préférence un polymère pris dans la famille du polypropylène (PP).

Si l'on souhaite un fil chirurgical résorbable, on choisira de préférence un polymère pris dans la famille de l'acide polylactique de forme L (PLLA).

De préférence, les fils unitaires sont des monofilaments, faisant un diamètre de 0,10 mm à 0,20 mm.

Ils sont suffisamment fins pour procurer la souplesse nécessaire au fil chirurgical. Lorsque le fil chirurgical est utilisé en comblement des rides ou pour remonter les tissus du visage qui s'affaissent, sa section finale occupée est de préférence compris entre 0,30 mm et 0,70 mm.

Les fils unitaires formant les tronçons de fil de blocage peuvent être choisis dans une famille de polymères ou de métaux, et/ou avec une section qui diffère des autres fils unitaires formant la succession continue de mailles. On peut en particulier obtenir un fil chirurgical qui n'est que partiellement résorbable, en choisissant par exemple des tronçons de fils de blocage résorbables et des fils formant la succession continue de mailles non résorbables. On peut par exemple augmenter l'accroche des projections en prenant pour les tronçons defils de blocage des monofilaments métalliques, plus rigides, notamment en acier inoxydable.

Dans une variante de réalisation, le fil chirurgical, prêt à l'usage, comprend unguide-fil formant un prolongement rigide à l'une ou à ses deux terminaisons. Ce guide-fil est destiné à faciliter la manipulation du fil chirurgical lors de sa fabrication et surtout lors du travail du chirurgien. En effet, le fil chirurgical peut n'être pas lui-même suffisamment rigide, notamment pour être enfilé dans de bonnes conditions dans les aiguilles creuses utilisées en chirurgie esthétique.

Le guide fil peut être un fil monofilament ou une aiguille chirurgicale longue sertie à l'une ou aux deux terminaisons du fil de suture.

De préférence, le guide-fil est un monofilament en polypropylène ayant une section, faisant par exemple de 0,20 mm à 0,70 mm, supérieure à celle des fils unitaires du fil chirurgical. La section du guide-fil est du même ordre que la section totale occupée par le fil chirurgical.

Dans une variante, un ensemble de fils chirurgicaux comprend au moins deux fils chirurgicaux selon la présente invention, qui sont reliés l'un à l'autre par au moins un tronçon commun de fil de blocage. Le dit tronçon de fil de blocage comporte deux extrémités libres, chacune se projetant sur au moins un desdits deux fils chirurgicaux. Dans le cas d'un ensemble ne comprenant que deux fils chirurgicaux, on obtient par exemple une configuration en forme de « X ». L'intersection entre les deux fils chirurgicaux crée une zone renforcée et soutenue selon les quatre directions différentes des branches du « X ». Les dites branches, telles que des portions de fil chirurgical, conservent éventuellement des projections s'étendant de part et d'autre hors de la section occupée par les successions continues de mailles les constituant. On peut faire varier la configuration du dit ensemble à l'infini. Par exemple, il est possible d'obtenir un ensemble ayant une configuration en forme d'étoile.

La présente invention a également pour objet un procédé de fabrication, spécialement conçu pour l'obtention du fil chirurgicale, précité qui comprend les opérations suivantes :
1) une opération de tricotage d'un panneau textile selon une armure déterminée de manière à ce que le dit panneau textile comporte des colonnes continues de mailles et des fils de trame assurant la liaison et le blocage des dites colonnes, dénommés fils de blocage, et
2) des opérations de découpe longitudinale et transversale du panneau textile, découpe longitudinale selon les intervalles entre deux colonnes de mailles adjacentes, provoquant la coupe des fils de trame et donc laformation des tronçons de fils de blocage et la séparation d'au moins une colonne continue de mailles, liées par les dits tronçons, constituant le fil chirurgical.

Le panneau textile peut être un tricot, connu sous l'appellation indémaillable, du type chaîne ou raschel.

Les fils de trame une fois découpés forment des tronçons de fils blocage dont les extrémités libres, dépassant plus ou moins de la section occupée par la colonne de mailles, forment les projections du fil chirurgical. Ainsi, chaque colonne de mailles comporte sur sa longueur une succession de projection selon une séquence qui est fonction de l'armure de tricotage.

La longueur des projections est déterminée par la distance à laquelle la découpe a lieu par rapport aux colonnes de mailles. En pratique, la découpe intervient à mi distance entre deux colonnes adjacentes et la longueur des projections est déterminée par l'armure de tricotage qui définit l'écartement entre deux colonnes de mailles. La découpe des fils de blocage est réalisée à l'aide de ciseaux électriques, par ultrasons ou tout autre moyen de découpe équivalent.

Dans une variante de réalisation, le procédé de fabrication comprend une étape de fixation d'un guide-fil à au moins l'une des terminaisons du dit fil chirurgical par un noeud, une soudure diélectrique (type ultrasons ou haute fréquences), collage, thermosoudure, sertissage ou tout autre moyen de fixation équivalent.

Dans une variante de réalisation, le procédé de fabrication comprend une étape ultérieure de thermo fixation au cours de laquelle au moins une portion d'un fil chirurgical, formé de fils unitaires en polymère, est éventuellement soumise à une contrainte mécanique transversale, dans des conditions de température, de pression et de durée déterminées en fonction de la nature du dit polymère notamment de sa température de transition vitreuse (Tg) et/ou sa température de fusion.

L'intensité de la dite contrainte mécanique transversale permet notamment de régler la direction et l'angle (α) d'inclinaison des projections sur la portion thermofixée. De la sorte, il est possible d'obtenir une ou plusieurs portions, disposées de façon déterminée et selon une longueur déterminée, sur le fil chirurgical ; par exemple plusieurs portions présentant des projections, inclinées dans des directions opposées ou non, et délimitant des angles (α) identiques ou différents avec l'axe longitudinal (XX') du fil.

Dans un mode de réalisation, l'étape de thermo fixation est mise en oeuvre en introduisant au moins une portion du fil chirurgical dans un tube de diamètre et longueur déterminés, puis en plaçant le dit tube et le fil chirurgical dans une étuve dans des conditions de température, de pression et de durée déterminées.

L'introduction du fil chirurgical dans le tube peut être facilitée par le guide fil.

C'est la valeur du rapport (R) entre le diamètre intérieur du tube et le diamètre qui correspond à la section totale du fil chirurgical, incluant les projections dépassant sur l'extérieur de la section occupée par la succession de mailles qui détermine l'intensité de la contrainte mécanique transversale qui est éventuellement appliquée.

La direction de l'inclinaison des projections est déterminée par le sens d'introduction du tube.

Si le tube a un diamètre intérieur légèrement supérieur ou égal à la section totale du fil chirurgical, la contrainte mécanique transversale appliquée est faible voire nulle. Si le tube a un diamètre intérieur inférieur à la section totale du fil chirurgical, les projections sont repoussées vers la colonne de mailles. L'inclinaison et l'angle formés par les projections sont ajustés et bloqués grâce à la thermo fixation.

Le tube peut être en matériau métallique ou en matériau polymère, résistant aux conditions opératoires de la thermo fixation.

Dans une variante, le procédé permet la fabrication d'un assemblage de fils chirurgicaux, ayant les caractéristiques de la présente invention. De manière particulière, entre deux découpes transversales séparées par une distance donnée correspondant à la hauteur de l'assemblage, on procède :
- à des découpes longitudinales continues sur toute la dite hauteur selon des intervalles non adjacents et
- à des découpes longitudinales partiellement interrompues selon les intervalles compris entre les dits intervalles non adjacents,
de manière à former un assemblage de fils chirurgicaux reliés par les fils de trame non coupés.

Dans le cas par exemple d'un ensemble de deux fils chirurgicaux les dites découpes longitudinales continues sont réalisées selon deux intervalles non adjacents séparés par un seul intervalle intercalaire, tandis que leur découpe longitudinale partielle est réalisée selon l'intervalle intercalaire, étant interrompue sur une certaine hauteur, de sorte que certains fils de trame ne sont pas coupés. Ces fils de trame non coupés se trouvent alors sur chaque fil chirurgical unitaire et réalisent la liaison entre les deux dits fils chirurgicaux.

De préférence, la hauteur entre deux découpes transversales est comprise entre 15 cm et 35 cm, et plus particulièrement égale à 23 cm.

La présente invention sera mieux comprise à la lecture de la description qui va être faite d'exemples de fil chirurgicale, notamment pour suturer et dans le domaine de la chirurgie esthétique, donnés à titre non limitatif.
- Les figures 1 et 3 sont des représentations schématiques de panneaux de tricot, du type chaîne à deux évolutions de trame.
- La figure 2 est une représentation schématique d'un fil chirurgical obtenu à partir du panneau de la figure 1.
- La figure 4 représente de façon schématique, vue de côté, un fil chirurgical de la figure 2, au cours de l'étape de thermo fixation sous contrainte mécanique transversale.

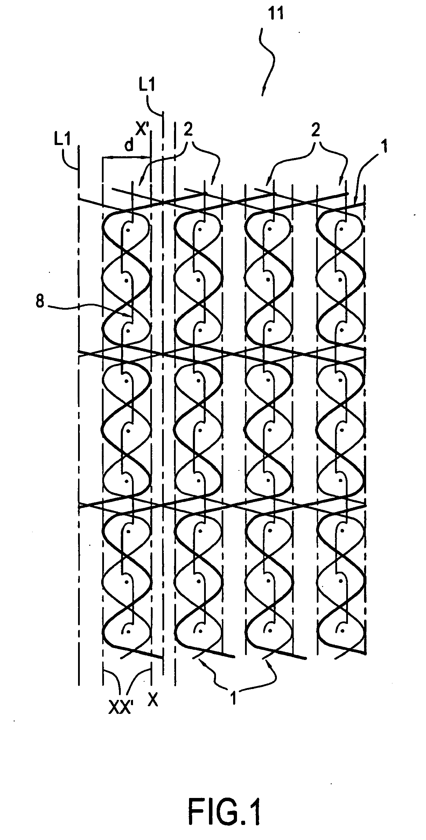

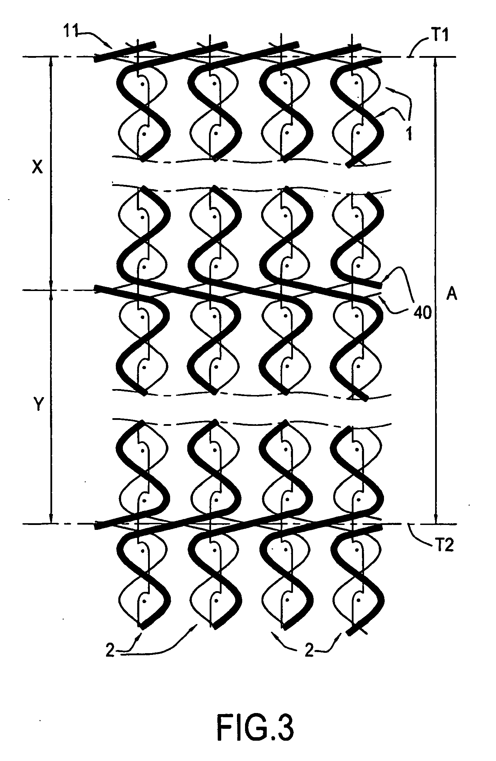

Par évolution de trame, on comprend le parcours suivi par le fil de trame dans un panneau textile tricoté 11. Chaque armure sur les figures 1 et 3 comporte trois types de fils unitaires, à savoir deux fils de trame « travaillant » avec un fil de chaîne tricotée sur une aiguille formant une colonne de mailles, appelée chaînette. Il y a autant d'aiguilles que de colonnes continues de mailles. Les fils de trame représentés sur les panneaux tricotés (11) dans les figures 1 et 3 travaillent sur une colonne de mailles puis sur la colonne de mailles adjacentes.

Les armures de tricotage représentées aux figures 1 et 3 sont citées à titre d'exemples, le fil chirurgical selon la présente invention peut être obtenu à partir de panneaux tricotés selon d'autres armures non représentées.

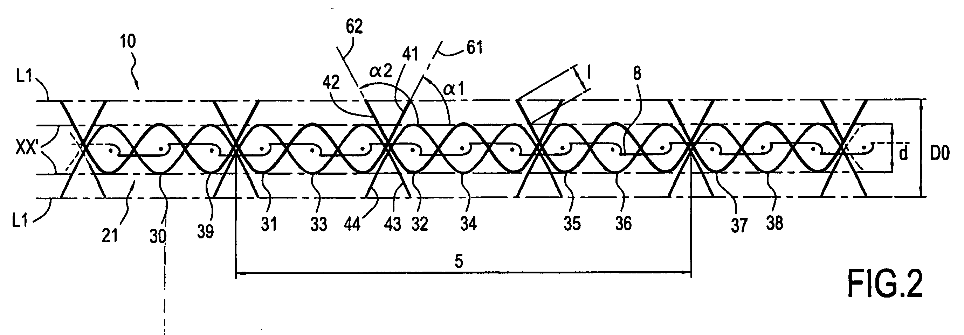

Dans l'exemple de la figure 1, pour obtenir le fil chirurgical 10 de la figure 2, les fils de trame 1 du panneau textile 11, obtenu par tricotage selon l'armure de la figure 1, sont découpés le long et entre deux colonnes continues de mailles 2 selon une ligne de découpe L1 parallèle aux axes longitudinaux XX'. On obtient ainsi le fil 10 de section DO qui est formé par une succession continue de mailles 2, et par des tronçons 31,32,33,34 de fils de trame, dont les extrémités libres dépassent des dites colonnes. Dans l'exemple des figures 1 et 2, il y a un groupe de quatre projections libres, dénommées projections 41,42,43,44 toutes les trois mailles. Sur la longueur 5 repérée à la figure 2, la colonne continue de mailles 2 est bloquée par six tronçons de fils de blocage 31,32,33,34,35,36, les tronçons 30, 39, 37, 38 ne bloquent pas le fil chirurgical 10 sur la longueur 5.

Les projections 41 et 42 forment deux angles α1 et α2 avec l'axe longitudinal XX' de la colonne continue de mailles 2. Sans thermo fixation, c'est l'armure de tricotage seule qui définit la valeur des angles α1 et α2. Dans l'exemple de la figure 2, α1 vaut 70°+/-30° et α2 vaut 110° +/-30°. La longueur (I) des projections est de préférence comprise dans l'intervalle [0 mm ; 5mm].

Dans un exemple précis de réalisation d'un fil chirurgical 10, tous les fils unitaires sont des monofilaments en polypropylène de diamètre de l'ordre de 0,10 mm. Le diamètre DO de la section finale du fil chirurgical 10, sans tension appliquée à l'une de ses terminaisons, est d'environ 0,30 mm.

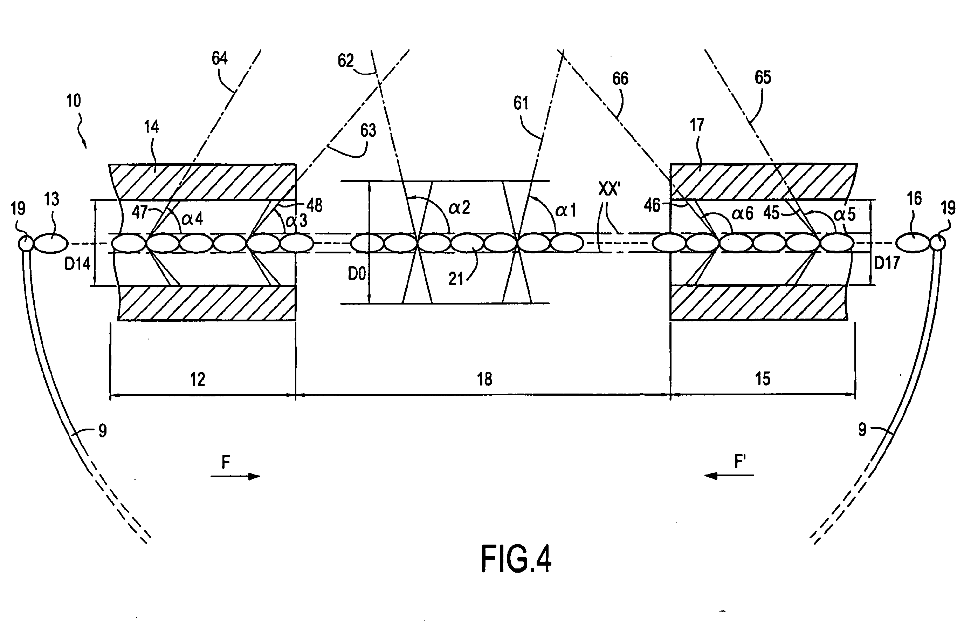

Afin de constituer un guide fil, on peut fixer un monofilament 9 par un noeud 19 (figure 4), par exemple en polypropylène de diamètre égal à 0,30 mm, aux deux terminaisons du fil chirurgical 10.

A la figure 2, les groupes de projections 41,42,43,44 le long de la colonne de mailles 2 ont une séquence égale à trois mailles. Il est possible de faire varier cette séquence afin de rapprocher ou d'espacer les projections. L'armure de tricotage représentée à la figure 3, représente des variantes d'armures de tricotage, de type chaîne ou raschel, à deux évolutions de fils trame permettant notamment de faire varier la dite séquence.

La zone A entre les axes T1 et T2 délimite le raccord d'armure. Le dessin ou l'armure de tricotage supportée par le raccord d'armure se reproduit sur le panneau textile 11 au-delà des axes T1 et T2.

Dans le raccord d'armure représenté à la figure 3, X et Y délimitent des intervalles constitués par un nombre donné de mailles sur la colonne de mailles 2 et se terminant par les groupes de quatre projections. A la figure 1, X et Y sont égales à trois mailles.
X et Y peuvent indépendamment prendre les valeurs suivantes :
X : 3-5-7-9-11-13-15-17-19-21-23-25-27-29-31
Y: 3-5-7-9-11-13-15-17-19-21-23-25-27-29-31

Il est possible de faire varier de façon déterminée les angles (α1) et (α2) lors d'une opération de thermo fixation. Cette opération est réalisée par exemple au moyen de tubes de diamètres internes déterminés. Les tubes sont dans un matériau polymère dont la température de fusion est supérieure à celle programmée dans l'étuve. Dans le cas d'un fil chirurgical en polypropylène, l'ensemble fil chirurgical pourvu d'un ou plusieurs des dits tubes est placée à l'étuve entre 100°C et 150°C, à pression atmosphérique pour une durée inférieure à 10 minutes. On introduit dans le sens de la flèche F' (figure 5) la portion 12 du fil de suture 10 à partir de la terminaison 13 dans un premier tube 14 et une autre portion 15 à partir de la terminaison 16 dans un second tube 17 selon la flèche F. Le premier tube 14 a un diamètre intérieur D14 10 inférieur à celui de la section DO occupée par le fil chirurgical 10 et le second tube 17 a un diamètre intérieur D17 également inférieur à celui de la section DO mais supérieur au diamètre D14 du premier tube 14.

Lors de l'introduction du fil dans le tube, les projections raclent la paroi interne du tube et fléchissent par rapport à leur partie initiale. Comme il apparaît sur la figure 5, adoptant une direction selon les axes 63 et 64.

A la figure 4, D17/DO > D14/DO, la contrainte mécanique transversale appliquée sur la portion 15 est moins importante que celle appliquée sur la portion 12. On obtient de la sorte, une portion 15 plus souple que la portion 12 rigidifiée. En outre, les angles (α5) et (α6) sont de préférence compris dans l'intervalle [90° ; 140°] et sont moins obtus par rapport à l'axe XX' que les angles (α3) et (α4) lesquels sont compris de préférence dans l'intervalle [15° 45°]. Les angles (α3) et (α4) sont sensiblement identiques mais non strictement égaux car les projections ne peuvent se confondre ainsi les axes 64 et 63 sont quasi parallèles. Il en est de même pour les angles (α5) et (α6) et des axes 66 et 65.

Si le rapport D14/DO est égal au rapport D17/DO, la contrainte mécanique transversale appliquée sur la première portion 12 est identique à celle appliquée sur la portion 15. On obtient ainsi deux angles (α3) et (α4) sensiblement égaux compris de préférence dans l'intervalle [15° ; 45°] dont les projections 47,48 sont inclinées dans le sens de la flèche F, opposé au sens de la flèche F' selon lequel sont inclinées les projections 45,46 formant des angles (α5) et (α6) sensiblement égaux compris de préférence dans l'intervalle [135° ; 165°]. En outre, la contrainte mécanique transversale appliquée sur les portions 12 et 15 étant identique, les angles (α3) et (α4) sont égaux en valeur absolue aux angles (α5) et (α6).

La portion 18 de la dite succession continue de mailles 2 n'est pas soumise à une contrainte mécanique transversale liée aux tubes mais subit les conditions de la thermo fixation puisque le fil chirurgical 10 est placé dans l'étuve en même temps que les tubes 14,17, la portion 18 est donc plus élastique grâce à la relaxation des polymères constituant le fil 10 et les projections conservent les angles (α1) et (α2) déterminés lors de la découpe des fils de trame par l'armure de tricotage.

Le fil chirurgical de la présente invention peut être utilisé dans de nombreuses applications, et notamment dans les domaines suivants, cités à titre non limitatif :
- pour suturer les tissus souples internes du corps humain, notamment les lèvres d'une plaie ;
- en chirurgie esthétique, par exemple dans le comblement des rides et/ou pour lutter contre la chute des tissus en remontant ces derniers en particulier la peau du visage, et plus particulièrement les tissus sur les joues et les paupières ;
- en chirurgie viscérale pour fixer la plaque de hernie lors de la réfection de la paroi abdominale ;
- en chirurgie de l'estomac (notamment au cours de la Nissen Fundoplication)
- en traitement des incontinences urinaires et des prolapsus génitaux urinaires pour remonter les organes pelviens.

## Revendications

1. Fil chirurgical (10) **caractérisé en ce qu'**il a une structure maillée, se présentant sous la forme d'une succession continue de mailles (2), bloquées par intermittence par des tronçons de fils de blocage (30-39).

2. Fil chirurgical (10) pour la chirurgie esthétique selon la revendication 1, **caractérisé en ce que** les dits tronçons de fils de blocage (30-39) ont leurs extrémités libres qui viennent en saillie au-delà, de façon séquentielle, de la section occupée d par la dite succession continue de mailles (2).

3. Fil chirurgical (10) selon, l'une ou l'autre des revendications 1 et 2, **caractérisé en ce qu'**il comporte au moins un premier ensemble des dites projections (12), dans lequel chaque projection est inclinée dans une même première direction (F) et éventuellement au moins un second ensemble (15) des dites projections, dans lequel chaque projection est inclinée dans une même seconde direction (F') opposée à la première.

4. Fil chirurgical (10) selon l'une des revendications 1 à 3, **caractérisé en ce que** la direction des dites projections forment des angles déterminés avec l'axe longitudinal (XX') du fil de préférence dans l'intervalle [15°; 45°].

5. Fil chirurgical (10) selon l'une quelconque des revendications précédentes, **caractérisé en ce qu'**il comprend des monofilaments en polypropylène (PP).

6. Fil chirurgical (10) selon l'une quelconque des revendications précédentes, **caractérisé en ce qu'**il comprend des monofilaments en PLLA.

7. Fil chirurgical (10) selon l'une quelconque des revendications précédentes, **caractérisé en ce qu'**il comprend des monofilaments ayant un diamètre compris dans l'intervalle [0,10 mm ; 0,20 mm].

8. Fil chirurgical (10) selon l'une quelconque des revendications précédentes, **caractérisé en ce qu'**il comprend un guide fil (9) formant un prolongement à l'une ou à ses deux dites terminaisons (13,16).

9. Fil chirurgical (10) selon la revendication 8, **caractérisé en ce que** le guide fil (9) comprend au moins un monofilament en polypropylène ayant un diamètre compris dans l'intervalle [0,20 mm ; 0,70 mm].

10. Assemblage de fils chirurgicaux (10) selon l'une quelconque des revendications précédentes, **caractérisé en ce qu'**il comprend au moins deux fils chirurgicaux (10) reliés l'un à l'autre par au moins un tronçon de fil de blocage commun (30-39).

11. Procédé de fabrication d'un fil chirurgical (10), **caractérisé en ce qu'**il comprend les opérations suivantes :
1) une opération de tricotage d'un panneau textile (11) selon une armure déterminée de manière à ce que le dit panneau textile comporte des colonnes continues de mailles (2) et des fils de trame assurant la liaison des dites colonnes, dénommés fils de blocage, et
2) des opérations de découpe longitudinale et transversale du panneau textile (11), découpe longitudinale selon les intervalles entre deux colonnes de mailles adjacentes, provoquant la coupe des dits fils de trame et donc la formation des tronçons de fils de blocage, et la séparation d'au moins une colonne continue de mailles, liées par les dits tronçons constituant le fil chirurgical (10).

12. Procédé de fabrication d'un fil chirurgical (10) selon la revendication 11, **caractérisé en ce qu'**il comprend une étape de fixation d'un guide fil (9) à au moins l'une des terminaisons (13,16) dudit fil chirurgical (10) notamment par un noeud (19), une soudure diélectrique (type ultrasons), collage, thermosoudure ou sertissage.

13. Procédé de fabrication d'un fil chirurgical (10) selon l'une ou l'autre des revendications 11 et 12, **caractérisé en ce qu'**il comprend une étape ultérieure de thermo fixation au cours de laquelle au moins une portion (12,15) du dit fil chirurgical (10), formé de fils unitaires en polymère, est éventuellement soumise à une contrainte mécanique transversale, dans des conditions de température, de pression et de durée déterminées, en fonction de la nature du dit polymère notamment de sa température de transition vitreuse (Tg) et/ou sa température de fusion.

14. Procédé de fabrication d'un fil chirurgical (10) selon la revendication 13, **caractérisé en ce que** l'étape de thermo fixation est mise en oeuvre en introduisant au moins une portion (12,15) du dit fil chirurgical dans un tube (14,17) de diamètre (D14,D17) et longueur déterminés puis en plaçant le dit tube (14,17) et le fil chirurgical (10) dans une étuve dans des conditions de température, de pression et de durée déterminées.

15. Procédé, selon la revendication 11, pour la fabrication d'un assemblage de fils chirurgicaux, **caractérisé en ce que**, entre deux découpes transversales séparées par une distance donnée correspondant à la hauteur des fils chirurgicaux de l'assemblage, on procède :
- à des découpes longitudinales continues sur toute la dite hauteur selon des intervalles non adjacents et
- à des découpes longitudinales partiellement interrompues selon les intervalles compris entre les dits intervalles non adjacents,
de manière à former un assemblage de fils chirurgicaux reliés par les fils de trame non coupés.

## Claims

1. Surgical thread (10) **characterized in that** it has a stitch structure, appearing as a continuous succession of stitches (2), intermittently blocked by sections of blocking threads (30-39).

2. Surgical thread (10) for cosmetic surgery according to claim 1, **characterized in that** said sections of blocking threads (30-39) have their free ends that will sequentially protrude out beyond the section d occupied by said continuous succession of stitches (2).

3. Surgical thread (10) according to either one of claims 1 and 2, **characterized in that** it includes at least one first set of said projections (12), wherein each projection is tilted in a same first direction (F), and optionally at least one second set (15) of said projections, wherein each projection is tilted in a same second direction (F') opposite to the first.

4. Surgical thread (10) according to any of claims 1 to 3, **characterized in that** the direction of said projections form determined angles with the longitudinal axis (XX') of the thread, preferably in the interval [15°; 45°].

5. Surgical thread (10) according to any of the preceding claims, **characterized in that** it comprises polypropylene (PP) monofilaments.

6. Surgical thread (10) according to any of the preceding claims, **characterized in that** it comprises PLLA monofilaments.

7. Surgical thread (10) according to any of the preceding claims, **characterized in that** it comprises monofilaments having a diameter comprised in the interval [0.10 mm; 0.20 mm].

8. Surgical thread (10) according to any of the preceding claims, **characterized in that** it comprises a guide thread (9) forming an extension to one or to its two said terminations (13, 16).

9. Surgical thread (10) according to claim 8, **characterized in that** the guide thread (9) comprises at least one polypropylene monofilament having a diameter comprised in the interval [0.20 mm; 0.70 mm].

10. An assembly of chirurgical threads (10) according to any of the preceding claims, **characterized in that** it comprises at least two surgical threads (10) connected to each other through at least one common section of blocking thread (30-39).

11. Method for making a surgical thread (10), **characterized in that it** comprises the following operations:
1) an operation for knitting a textile panel (11) according to a weave determined so that said textile panel includes continuous columns of stitches (2) and weft threads providing the connection of said columns, so-called blocking threads, and
2) operations for longitudinally and transversely cutting out the textile panel (11), longitudinally cutting along the intervals between both columns of at least adjacent stitches, and causing said weft threads to be cut and therefore formation of the sections of blocking threads, and separation of at least one continuous column of stitches, connected by said sections forming the surgical thread (10).

12. Method for making a surgical thread (10) according to claim 11, **characterized in that** it comprises a step for attaching a guide thread (9) to at least one of the terminations (13, 16) of said surgical thread (10) notably by a knot (19), a dielectric (ultrasonic type) weld, adhesive bonding, thermowelding or crimping.

13. Method for making a surgical thread (10) according to either one of claims 11 and 12, **characterized in that** it comprises a subsequent step of heat fixation during which at least one portion (12, 15) of said surgical thread (10), formed with unitary polymeric threads, is optionally subject to a transverse mechanical stress, under determined conditions of temperature, pressure and duration, depending on the nature of said polymer, notably on its glassy transition temperature (TG) and/or its melting temperature.

14. Method for making a surgical thread (10) according to claim 13, **characterized in that** the heat fixation step is applied by introducing at least one portion (12, 15) of said surgical thread into a tube (14, 17) of determined diameter (D14, D17) and length and then by placing said tube (14, 17) and the surgical thread (10) in an oven under determined conditions of temperature, pressure and duration.

15. Method according to claim 11, for making an assembly of surgical threads, **characterized in that**, between two transverse cuts separated by a given distance corresponding to the height of the surgical threads of the assembly, it is proceeded with:
- longitudinal continuous cuts over the whole of said height along non-adjacent intervals and
- longitudinal partly interrupted cuts along the intervals comprised between said non-adjacent intervals,
so as to form an assembly of surgical threads connected through non-cut weft threads.

## Patentansprüche

1. Chirurgischer Faden (10), **dadurch gekennzeichnet, daß** er eine Maschenstruktur aufweist, die in Form einer ununterbrochenen Folge von Maschen (2) vorliegt, die durch Blockierfadenabschnitte (30 39) intermittierend blockiert sind.

2. Chirurgischer Faden (10) für die ästhetische Chirurgie nach Anspruch 1, **dadurch gekennzeichnet, daß** die Blockierfadenabschnitte (30 - 39) freie Enden aufweisen, die über den durch die ununterbrochene Folge von Maschen (2) eingenommenen Querschnitt d sequentiell hinausragen.

3. Chirurgischer Faden (10) nach dem einen oder dem anderen der Ansprüche 1 und 2, **dadurch gekennzeichnet, daß** er wenigstens eine erste Einheit der Vorsprünge (12) umfaßt, bei der jeder Vorsprung in einer gleichen ersten Richtung (F) geneigt ist, sowie eventuell wenigstens eine zweite Einheit (15) der Vorsprünge, bei der jeder Vorsprung in einer gleichen zweiten, zur ersten Richtung entgegengesetzten Richtung (F') geneigt ist.

4. Chirurgischer Faden (10) nach einem der Ansprüche 1 bis 3, **dadurch gekennzeichnet, daß** die Richtung der Vorsprünge mit der Längsachse (XX') des Fadens bestimmte Winkel einschließt, vorzugsweise im Bereich [15°; 45°].

5. Chirurgischer Faden (10) nach einem der vorhergehenden Ansprüche, **dadurch gekennzeichnet, daß** er Monofilamente aus Polypropylen (PP) aufweist.

6. Chirurgischer Faden (10) nach einem der vorhergehenden Ansprüche, **dadurch gekennzeichnet, daß** er Monofilamente aus PLLA aufweist.

7. Chirurgischer Faden (10) nach einem der vorhergehenden Ansprüche, **dadurch gekennzeichnet, daß** er Monofilamente mit einem Durchmesser im Bereich [0,10 mm; 0,20 mm] aufweist.

8. Chirurgischer Faden (10) nach einem der vorhergehenden Ansprüche, **dadurch gekennzeichnet, daß** er einen Fadenführer (9) aufweist, der an einem seiner oder an seinen beiden Enden (13, 16) eine Verlängerung bildet.

9. Chirurgischer Faden (10) nach Anspruch 8, **dadurch gekennzeichnet, daß** der Fadenführer (9) wenigstens ein Polypropylen-Monofilament mit einem Durchmesser im Bereich [0,20 mm; 0,70 mm] aufweist.

10. Verbindung von chirurgischen Fäden (10) nach einem der vorhergehenden Ansprüche, **dadurch gekennzeichnet, daß** sie wenigstens zwei chirurgische Fäden (10) umfaßt, die durch wenigstens einen gemeinsamen Blockierfadenabschnitt (30 - 39) miteinander verbunden sind.

11. Verfahren zur Herstellung eines chirurgischen Fadens (10), **dadurch gekennzeichnet, daß** es die folgenden Schritte umfaßt:
1) einen Schritt zum Wirken einer Textilfläche (11) mit einer bestimmten Bindung, so daß die Textilfläche durchgehende Reihen von Maschen (2) sowie die Verbindung der Reihen sicherstellende Schußfäden, die als Blockierfäden bezeichnet werden, aufweist, und
2) Schritte zum Längs- und Querschneiden der Textilfläche (11), Längsschneiden entlang der Zwischenräume zwischen zwei benachbarten Maschenreihen, was das Zerschneiden der Schußfäden und folglich die Bildung der Blockierfadenabschnitte sowie die Trennung wenigstens einer den chirurgischen Faden (10) bildenden durchgehenden Reihe von durch die Abschnitte verbundenen Maschen bewirkt.

12. Verfahren zur Herstellung eines chirurgischen Fadens (10) nach Anspruch 11, **dadurch gekennzeichnet, daß** es einen Schritt zum Fixieren eines Fadenführers (9) an wenigstens einem der Enden (13, 16) des chirurgischen Fadens (10), insbesondere mittels eines Knotens (19), durch eine dielektrische Schweißung (Typ Ultraschall), Kleben, Heißsiegeln oder Crimpen umfaßt.

13. Verfahren zur Herstellung eines chirurgischen Fadens (10) nach dem einen oder dem anderen der Ansprüche 11 und 12, **dadurch gekennzeichnet, daß** es einen nachträglichen Schritt einer Thermofixierung umfaßt, im Laufe dessen wenigstens ein Abschnitt (12, 15) des chirurgischen Fadens (10), der von Polymereinzelfäden gebildet ist, unter bestimmten Temperatur-, Druck- und Dauerbedingungen, in Abhängigkeit der Art des Polymers, insbesondere seiner Glasübergangstemperatur (Tg) und/oder seiner Schmelztemperatur, eventuell einer mechanischen Querbeanspruchung ausgesetzt wird.

14. Verfahren zur Herstellung eines chirurgischen Fadens (10) nach Anspruch 13, **dadurch gekennzeichnet, daß** der Schritt der Thermofixierung **dadurch** vollzogen wird, daß wenigstens ein Abschnitt (12, 15) des chirurgischen Fadens in ein Rohr (14, 17) mit bestimmtem Durchmesser (D14, D17) und bestimmter Länge eingeführt wird, anschließend das Rohr (14, 17) und der chirurgische Faden (10) unter bestimmten Temperatur-, Druck- und Dauerbedingungen in einen Trockenofen eingebracht werden.

15. Verfahren nach Anspruch 11, für die Herstellung einer Verbindung von chirurgischen Fäden, **dadurch gekennzeichnet, daß** zwischen zwei Querschnitten, die durch einen vorgegebenen, der Höhe der chirurgischen Fäden der Verbindung entsprechenden Abstand getrennt sind, folgendes durchgeführt wird:
- durchgehende Längsschnitte über die gesamte Höhe entlang nicht benachbarter Zwischenräume, und
- teilweise unterbrochene Längsschnitte entlang der zwischen den nicht benachbarten Zwischenräumen befindlichen Zwischenräume,
so daß eine Verbindung von chirurgischen Fäden, die durch die nicht zerschnittenen Schußfäden verbunden sind, gebildet wird.
